# EUROPEAN PATENT APPLICATION

(11) **EP 1 498 423 A1**
(43) Date of publication of application: **19.01.2005**
(21) Application number: 03077243.8
(22) Date of filing: 16.07.2003
(51) Int. Cl.: C07K 14/46

(54) **Regulatory region for Xenopus Lef-1 expression and its use**

(71) Applicant: Koninklijke Nederlandse Akademie van Wetenschappen, 1011 JV Amsterdam (NL)
(72) Inventor: Destree, Olivier Hubert Joseph, 1381 VV Weesp (NL); Spieker, Nicole, 3515 GV Utrecht (NL)
(74) Representative: Prins, Adrianus Willem, Mr. Ir.

(57) **Abstract**

In the present invention a regulatory sequence directing *Lef*-1 expression in *Xenopus* was found and used to find compounds capable of altering the expression characteristics of *Lef-1* both *in vitro* and *in vivo*. The sequence can be used to drive expression of a heterologous gene of interest and confer on that gene of interest an expression characeristic of *Lef-1*. The invention further provides the identification of a non-viral internal ribosomal entry site derived from the 5'untranslated region of *Lef*-1.

## Description

The invention relates to the fields of biology and medicine. The invention in particular relates to the selection of compounds that are capable of modulating transcription and translation of a *Lef-1* gene in animals and humans.

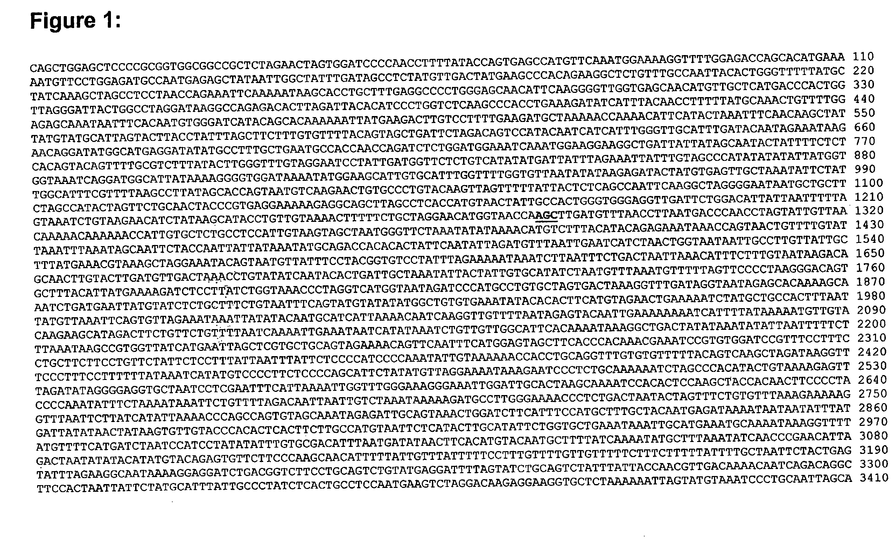

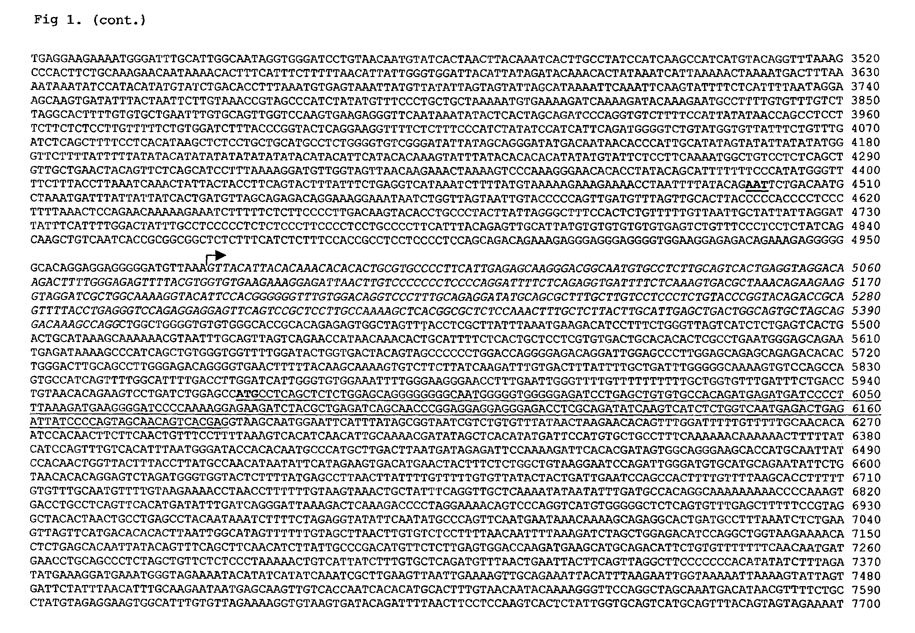

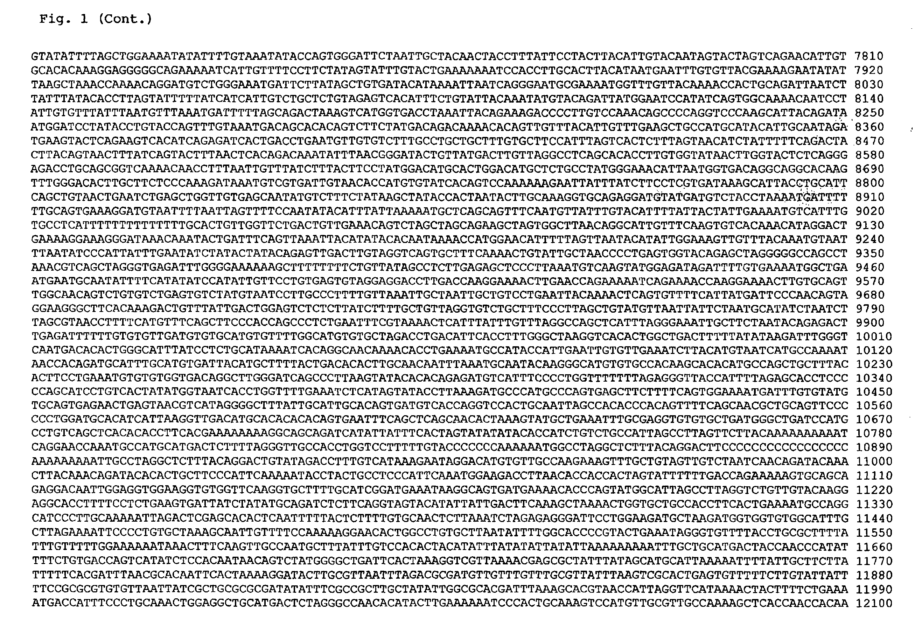

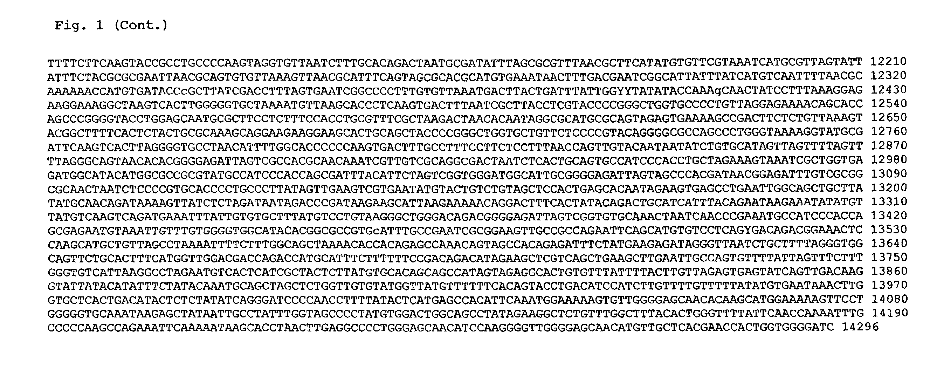

Lef-1 belongs to the Tcf/Lef family of transcription factors that function as mediators in the canonical Wnt-signalling cascade (Travis et al., 1991). *Lef-1* is involved in many different processes in a cell. Expression of *XLef-1* is differentially regulated in time and place during development (Molenaar et al., 1998). In the present invention we have identified a regulatory sequence capable of at least partially mimicking this expression. Said regulatory sequence is capable of conferring expression capabilities of an endogenous *Xlef-1* gene onto a variety of different genes. The sequences can be added to a minimal TATAA-box or other minimal transcription regulatory elements. This property can be used to generate cells having a foreign and/or endogenous gene provided with an *XLef-*like expression pattern. Such cells are a useful tool for determining means for interfering with endogenous *Lef-1* expression. Such means can then subsequently be used to manipulate the function of *Lef-1* and thus alter the development and/or function of cells having the *Lef-1* gene and/or their progeny. The invention therefore provides an isolated or recombinant nucleic acid comprising a regulatory sequence of a Xenopus *Lef-1* gene or a functional part, derivative and/or analogue thereof. A provided regulatory sequence is located in a nucleotide region of 3696 nucleotides located upstream of the transcription start site of said *Xenopus Lef-1* gene, as depicted in figure 1A, or a functional part, derivative and/or analogue thereof. The region may be expanded to a 4974 nucleotide region located upstream of the transcription start site of said *Xenopus Lef-1* gene, as depicted in figure 1, or a functional part, derivative and/or analogue thereof. The mentioned regulatory sequences allow tissue specific regulation of *XLef-1.* A minimal promoter region for *XtLef-1 (Xenopus tropicalis)* was determined by 5' deletion analysis in transient and stable reporter assays in *X. laevis* embryos. 5' sequences were fused to a GFP reporter. A 3696 bp *X. tropicalis Lef-1* 5' flanking region (plus 428 bp of UTR) was identified that mimics the endogenous *XLef-1* expression pattern *in vivo* both in *X. laevis* and *X*. *tropicalis.* This region can be extended to include sequences downstream of the *XLef-*1 promoter. In a particularly preferred embodiment the regulatory region further comprises a 994 nucleotide untranslated region, derivative and/or analogue and/or parts thereof. In the latter embodiment a closer regulation compared to the endogenous *XLef-1* is obtained. Furthermore translational control present in the 994 5' untranslated region, the exon 1 and part of the intron 1 of *XLef-1,* is included in the expression pattern of the produced RNA. Within the 5' region of 4974 or 3696 nucleotides, both activating and repressing elements were shown to be present controlling specific expression in (amongst others) the ventricle of the heart and outflow tract, in the developing inner ear including the endolymphatic sac, in the pituitary, pineal gland, lens and forebrain. The invention therefore provides an isolated and/or recombinant nucleic acid according to the invention wherein said 5' region regulatory sequence comprises a functional deletion of an activator sequence, wherein said activator sequence in the context of said regulatory sequence is capable of activating expression of an operably linked of *Xenopus Lef-1* gene and/or a gene of interest in the inner ear, the pituitary gland and/or the pineal gland, limb buds, lateral line system, epidermal hair cells, proctodeum and notochord. By functional deletion is meant an at least partial cis-acting deletion of the (activator) function. This can be achieved through a variety of different means known to the person skilled in the art, for instance but not limited to, deletion of the respective sequences from the regulatory sequence. Other means include, insertion of nucleotides within the region thereby at least in part inactivating the (activator) sequence. Further means include nucleotide substitutions thereby at least in part inactivating the (activator) sequence. The activator is comprised in a region that, in the sequence of Figure 1, is located between 4497 to 5402. The invention thus further provides said regulatory region wherein nucleotides 4497 to 5402 of figure 1, have been functionally deleted. In part of the regulatory sequence of the invention a repressor sequence was determined. This repressor sequence was found to direct repression of expression of the regulator sequence in, at least, the forebrain and/or lens. Interference with this repressor sequence can thus provide for expression of, for instance, endogenous *Lef-1* in cells wherein it is normally not expressed thereby providing a means for activating a wnt-signalling pathway in the mentioned cells. The present invention also provides means for interfering with this repressor sequence in that it provides a method for determining whether a compound is capable of interfering with a regulatory sequence of the invention. The compound may be tested for activity that mimics the expression pattern of a regulatory sequence of the invention from which the repressor sequence has been functionally deleted. To this end the invention provides an isolated and/or recombinant nucleic acid according to the invention, wherein said regulatory sequence comprises a functional deletion of a repressor sequence capable of repressing expression of *Xenopus Lef-1* and/or said gene of interest in the forebrain, pituitary and/or lens. The functional deletion can be achieved in much the same way as mentioned above for the functional deletion of the activator sequence. The repressor sequence is comprised in a region between 1278 to 4497 of the regulator sequence as depicted in figure 1. Thus further provided is an isolated nucleic acid as depicted in figure 1, wherein nucleotides 1278 to 4497 have been functionally deleted. The various implementations of the regulatory sequence of the invention can of course also be used as a promoter element to confer the mentioned expression (pattern) onto a gene of interest in one or more different cells or cell types. For instance by introducing the gene of interest operably linked to the regulatory sequence into the target cell(s) or precursor thereof.

Other means of conferring this expression pattern are not very often used but are also possible. For instance operably linkage of the regulatory sequence to an endogenous gene of interest, or visa versa operably linkage of the gene of interest to a regulatory region already present in the genome. This can be done for instance through homologous recombination. Thus the invention provides a method for regulating expression of a gene of interest in a cell comprising providing said cell with said gene of interest in operable linkage with a regulatory sequence of the invention, and culturing said cell to allow expression of said gene of interest. The cell can be any cell but is preferably a *Xenopus* cell. Using cells *in vitro* it is possible to design high througput systems for the screening of compounds (for example, Trumbull et al., 2003, Schnizler et al., 2003) that modulate the expression characteristics conferred by a regulatory sequence of the invention. In preferred embodiment the cell is *a Xenopus* germ-line cell, wherein the method further comprises producing a transgenic *Xenopus* animal and/or embryo from said cell. This allows the analysis of multiple expression characteristics of the introduced regulatory sequence, and thereby allows the simultaneous determination of the effect of a compound on multiple expression characteristics. In a preferred embodiment said *Xenopus* animal, embryo and/or cell is *Xenopus tropicalis or Xenopus laevis.*

The invention further provides the use of a 5' regulatory region of *Lef-1* from a *non-Xenopus* species in a *Xenopus* cell.

As mentioned above, one of the objectives of the present invention is the identification of one or more compounds capable of affecting the regulatory characteristics of a regulatory sequence of the invention. The utility of such a compound is manifold and includes the selection from such compound of candidate medicaments for the treatment of diseases. Considering that the wnt-signalling pathway is involved in many cellular (developmental) processes in the body of a subject, it is perceived that the mentioned medicaments will include medicaments capable of reprogramming one or more cells in the bodyof a subject treated with the medicament, thereby allowing such cells to at least in part regenerate and thereby at least ameliorate the disease.

Thus the invention provides a method of the invention, further comprising providing said cell and or transgenic animal/embryo with a compound and determining whether expression of said gene of interest is affected by said compound. The gene of interest can be any nucleic acid sequence, including *Lef-1.* The gene of interest is preferably a heterologous gene, preferably a marker gene, for which easy detection means are available such as but not limited to GFP. In a non-limiting example the compound comprises a metal ion, preferably a lithium ion. Using LiCl treatment we were able to manipulate expression of the 5402 and 4124 promoter fragment in tail regeneration experiments. In another preferred embodiment the compound comprises a proteinaceous molecule or a nucleic acid. A nucleic acid can be RNA or DNA and may comprises anti-sense molecules capable of at least in part inhibiting the activity of an element of a regulatory sequence of the invention. The compound may comprise a functional equivalent of nucleic acid when the equivalent comprises the same activity in kind as the corresponding nucleic acid. The amount of the activity does not have to be the same. A suitable but non-limiting equivalent is PNA (a mimic of nucleic acids in which the phosphodiester backbone has been replaced by a pseudo-peptide chain). A proteinaceous molecule is a molecule comprising at least two amino acids or equivalents thereof in peptidic linkage with each other. Preferably, the proteinaceous molecule comprises at least 10 and more preferably at least 20 amino acids or equivalents thereof in peptidic linkage with each other. The proteinaceous molecule may act directly on a regulatory sequence of the invention but may also act through one or more proteinaceous molecules that act on said regulatory sequence. In a preferred embodiment said cell is provided with said proteinaceous molecule by providing said cell with a nucleic acid encoding said proteinaceous molecule.

In another aspect the invention provides a method for directing translation of an mRNA in a cell comprising providing said mRNA with the 5' untranslated region of a *Lef-1* gene or a functional part, derivative and/or analogue thereof. The 5'untranslated region may be derived from any *Lef-1* gene, but is preferably obtained from *Xenopus.* In the present invention it has been found that this region contains an internal ribosomal entry site (IRES). The sequence is capable of attracting ribosomes and allow them to initiate translation of a down-stream coding region irrespective of the presence of an upstream coding region on the same mRNA. Thus in a preferred embodiment said mRNA comprises at least two coding regions. Preferably, said 5' untranslated region is located downstream from the translation start site of the coding region closest to the cap-site of the mRNA. The IRES is preferably used to generate a bi- or pluri-cistronic mRNA in a higher eukaryote. The IRES derived from the mentioned 5'region of *Lef-1* is capable of most if not all the functions of a viral IRES in a bi-or multicistronic RNA though it may involve a different pathway and result in different amount of such function.

Thus the invention further provides an isolated and/or recombinant nucleic acid comprising the 5' untranslated region of a *Lef-1* gene or a functional part, derivative and/or analogue thereof. Preferably a *Xenopus* 5' untranslated region. The region is not particularly species specific and the *Xenopus* region functions as an IRES also in mammalian cells. The 5' untranslated region of a *Lef-1* gene preferably further comprises at least one coding region for a gene product. Preferably said nucleic acid encodes a poly-cistronic mRNA.

Further provided are an expression vector comprising an isolated and/or recombinant nucleic acid according to the invention and a cell comprising an isolated and/or recombinant nucleic acid according to the invention and/or an expression vector according the invention.

A functional part, derivative and/or analogue of a regulatory sequence of the invention comprises the same expression characteristics in kind as a regulatory sequence of the invention, though not necessarily in amount. The derivative and/or analogue do not include the similar region from genuses other than *Xenopus.* However, simple parts, substitutions, fusions and alterations of a *Xenopus* sequence having the same activity in kind, not necessarily in amount are included. A functional part of a regulatory sequence of the invention is at least the mentioned activator or repressor in the regulatory sequence. Said particular functional parts may also be obtained from other species including a human *Lef-1* regulatory sequence. Such parts from other genuses than *Xenopus* can be found through sequence homology and functional activity for instance by placing them in a regulatory region and verifying activity in a Xenopus. Preferably, the functional part is derived from a species of the *Xenopus* genus. A functional part comprises at least 30 nucleotides.

A functional part, derivative and/or analogue of a *Lef-1* IRES comprises the same activity as the *Xenopus Lef-1* IRES in kind, not necessarily in amount. Preferably, the functional part, derivative and/or analogue is derived from *Xenopus.* A part comprises at least 30 nucleotides and preferably at least 150 nucleotides. The derivative and/or analogue can be generated in various ways including but not limited to nucleotide substitution, insertion, deletion and or modification.

### Experimental Procedures

### Cloning of XtLef-1 and start site determination.

A Lambda FIX-II *Xenopus tropicalis* genomic library (constructed by Lisa Brunet and Richard Harland) (Chae et al., 2002) was screened using a probe spanning exon 1 from *Xenopus laevis Lef-1* (Molenaar et al., 1998). Two positive inserts were analyzed by restriction enzyme mapping and shotgun sequencing. All sequence reactions were done with the ABI prism 377 using the BigDye Ready Reaction system (Perkin Elmer). Transcription start sites were determined by sequencing the inserts isolated from twenty single colonies obtained from a 5' RACE with the GeneRacer kit (Invitrogen). As a gene-specific reverse primer we used 5'-acc-aga-gat-gac-ttg-ata-tct-gcg-agg-3'. RNA was isolated from stage 10.5 *X. tropicalis* embryos using the Roche/Boehringer TriPure RNA isolation kit.

### Embryo manipulation and microinjection.

Methods of egg collection, fertilization and embryo culture were as described (Gao et al., 1994). Developmental stages were assigned according to Nieuwkoop and Faber (1994).

### Transgenesis in X. laevis

The HBgl4.5XtLef-1::GFP construct was generated by cloning a 4124 bp fragment into the pEGFP-1 plasmid (CLONTECH). The EBgl920XtLef-1::GFP construct was generated by cloning a 905 bp fragment into the pEGFP-1 plasmid (CLONTECH). Plasmid DNA was linearized with *SacI* and *Afl*II and purified using a Qiaex II Gel Extraction Kit (Qiagen) with final elution in water. Transgenic*X*. *laevis* embryos were produced using restriction enzyme-mediated integration (REMI) as described (Kroll and Amaya, 1996). The sperm nuclei were decondensed with 2.5 µl of high-speed oocyte extract. *Afl*II was used to induce double strand breaks in the sperm chromatin. Normal embryos were selected and cultured in 0.1× MMR at 16°C until stage 35/36 after which culture was continued in tap water. Transgenic *X*. *tropicalis* embryos were produced using a modified protocol as described by (Sparrow et al., 2000).

### In situ hybridization.

Whole mount *in situ* hybridization was performed as described by Harland (Harland, 1991). To generate a probe for GFP, the GFP-CS2 plasmid was linearized with *Bam*HI. The *XLef-1* cDNA in pBluescript SK was linearized with *Nco*I. Antisense RNA was obtained using T7 polymerase.

### Results

### Cloning of genomic sequences of the Lef-1 gene from Xenopus tropicalis.

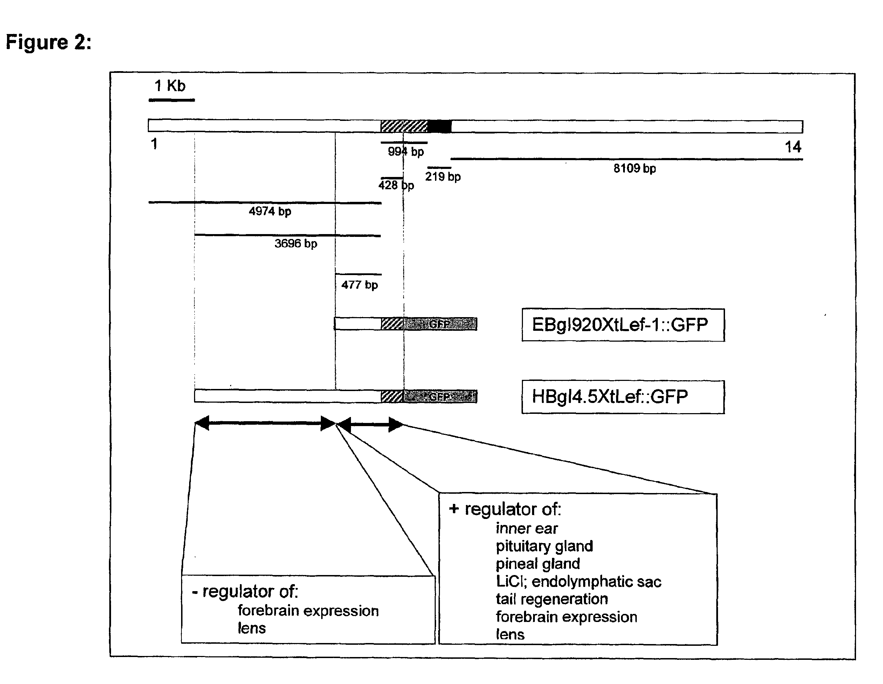

A *Xenopus tropicalis* genomic library was screened with a probe only containing exon 1 sequences of *Lef-1* from *X*. *laevis* (Molenaar et al., 1998). Two overlapping DNA fragments of 12-14 Kb were isolated and the complete sequence was determined (Figure 1). The start site of transcription was determined by 5' RACE (Figure 1 and 2).

### Regulation of the XtLef-1 promoter using a transgenic reporter assay in X. laevis and X. tropicalis.

To study regulation of the *X. tropicalis Lef-1* promoter in a chromatin context we used transgenic *X. laevis* and *X. tropicalis.* A linearized 4124 bp 5' fragment (Figure 2) including the promoter fused to a GFP reporter was injected together with sperm nuclei in unfertilized eggs. After selection at the four-cell stage, embryos were cultured and analyzed up to 4 weeks of development.

At embryonic stage 22, *in situ* analysis of the GFP expression revealed a positive signal in the midbrain and other sections of the CNS including the eye, in the branchial arches and in neural crest cells, in the dorsal part of the ear vesicles and in the somites. This pattern overlaps endogenous *Lef-1* expression.

At embryonic stage 32, endogenous expression of *Lef-1* is found in the midbrain, pronephros, somites, branchial arches, dorsal part of the eye and the developing ear. 4124bp of upstream sequences of *XtLef-1* (construct named HBgl4.5XtLef1::GFP) drive GFP expression in a similar fashion.

In two-week-old tadpoles, GFP expression was seen in the mesenchyme and derivatives in the head, branchial arches and subsequently in the gills, in the ventricle of the heart and the outflow tract. Also, expression was detected in specific sections of the branchial arch arteries, in specific regions of the telencephalon, in the eyes, the pituitary, the mesencephalon, in the epithelium of the otic vesicles. In four week old tadpoles, expression was found in specific sections of the inner ear, in the notochord, in specific domains of the somites, in the lateral plate mesoderm and mesoderm around the proctodeum, in the mesenchyme of the anterior and posterior limb buds as well as in the apical epidermal ridge of the limb buds and in the tailbud as well as in hair cells of the epidermis and the lateral line system.

Next, we made a 5' deletion of the promoter construct resulting in 905 bp of upstream sequences (Figure 2). We compared expression of this construct (Ebgl920Lef-1::GFP) to the HBgl4.5XtLef-1::GFP construct. Both constructs showed expression in most of the above-mentioned tissues and organs, however also several reproducible differences were detected. At tadpole stages (2-4 weeks), the EBgl920 promoter construct showed expression in the forebrain and lens, whereas the HBgl4.5 promoter was silent in these structures. The Ebgl920 promoter showed low or no expression in the inner ear, in the pituitary gland and in the pineal gland.

The expression patterns found with these constructs in transgenic *X*. *laevis* were also found in transgenic *X. tropicalis.*

### Manipulation of the XtLef-1 promoter using a transgenic reporter assay in X. laevis and X. tropicalis.

Because endogenous *XLef-1* and HBgl4.5XtLef-1::GFP are expressed in the growing tailbud but not in the tip of the definitive tail we tested if expression of HBglXtLef-1::GFP is induced during tail regeneration. Preliminary experiments show that HBglXtLef-1::GFP expression was up-regulated in the most distal part of the notochord in the original tail stump and in the epidermis within 6 hours after tail amputation. During regeneration of the tail XtLef-1::GFP remained strongly active in the stump part of the notochord as well as in the outgrowing newly formed notochord for at least one week. The therapeutic potential of stem cells has led to a renewed interest in classical models of regeneration (reviewed in (Tanaka, 2003)). Our construct can be used for this purpose.

At early tadpole stage, the HBgl4.5XtLef-1::GFP transgene is very active in specific domains of the inner ear, including the maculae in the ampullae of the semicircular canals and in the endolymphatic sac. During later tadpole stages activity in the endolymphatic sac and maculae fades away. Because *Lef-1* has been reported to be autoregulatory under certain circumstances (Filali et al., 2002) we tested the effects of lithium on HBgl4.5XtLef-1::GFP expression in late stage tadpoles. Lithium has numerous physiological targets and can affect Tcf/Lef target gene expression through inhibition of GSK-3 (Klein and Melton, 1996). Preliminary experiments show that lithium treatment (5 min, 20 mMol) induces the HBgl4.5XtLef-1::GFP transgene in the endolymphatic sac within 6 hours. These results suggest that the *XtLef-1* promoter is reactive to the canonical wnt signaling pathway through GSK3. HBgl4.5XtLef-1::GFP constructs containing different upstream regions will be used to test whether potential Tcf/Lef binding sites present in the 5' UTR and upstream sequence are functional in this respect. In analogy to the above described test for lithium responsiveness, expression of the transgene in the endolymphatic sac (and maculae) can be used to test the effects of small molecules, peptides hormones, and other signaling molecules including proteins into the membranous labyrinth. In these experiments, which can be performed in high throughput, the response in the "testear' can be directly compared to the response in the other ear as an internal control.

### Reference List

Chae,J., Zimmerman,L.B., and Grainger,R.M. (2002). Inducible control of tissue-specific transgene expression in Xenopus tropicalis transgenic lines. Mech. Dev. *117*,235-241.

Filali,M., Cheng,N., Abbott,D., Leontiev,V., and Engelhardt,J.F. (2002). Wnt-3A/beta-catenin signaling induces transcription from the LEF-1 promoter. J. Biol. Chem. *277*, 33398-33410.

Gao,X., Kuiken,G.A., Baarends,W.M., Koster,J.G., and Destree,O.H. (1994). Characterization of a functional promoter for the Xenopus wnt-1 gene on vivo. Oncogene *9*, 573-581.

Harland,R.M. (1991). In situ hybridization: an improved whole-mount method for Xenopus embryos. Methods Cell Biol. *36*, 685-695.

Klein,P.S. and Melton,D.A. (1996). A molecular mechanism for the effect of lithium on development. Proc. Natl. Acad. Sci. U. S. A *93,* 8455-8459.

Kroll,K.L. and Amaya,E. (1996). Transgenic Xenopus embryos from sperm nuclear transplantations reveal FGF signaling requirements during gastrulation. Development *122,* 3173-3183.

Molenaar,M., Roose,J., Peterson,J., Venanzi,S., Clevers,H., and Destree,O. (1998). Differential expression of the HMG box transcription factors XTcf-3 and XLef-1 during early xenopus development. Mech. Dev. *75,* 151-154.

Nieuwkoop PD Faber J. 1994. Normal table of *Xenopus laevis* (Daudin). Garland publishing , inc. New York and London.

Schnizler,K., Kuster,M., Methfessel,C., Fejtl,M. (2003). The roboocyte: automated cDNA/mRNA injection and subsequent TEVC recording in Xenopus oocytes in 96-well microtiter plates. Receptor Channels 9, 41-48.

Sparrow,D.B., Latinkic,B., and Mohun,T.J. (2000). A simplified method of generating transgenic Xenopus. Nucleic Acids Res. 28, E12.
Tanaka,E.M. (2003). Regeneration. If They Can Do It, Why Can't We? Cell *113,* 559-562.

Travis,A., Amsterdam,A., Belanger,C., and Grosschedl,R. (1991). LEF-1, a gene encoding a lymphoid-specific protein with an HMG domain, regulates T-cell receptor alpha enhancer function [corrected]. Genes Dev. *5*, 880-894.

Trumbell,J.D, Maslana,E.S., McKenna,D.G., Nemcek,T.A., Miforatos,W., Pan,J.Y., Parihar,A.S., Shieh,C.C., Wilkins,J.A., Briggs,C.A., Bertrand,D. (2003). High throughput electrophysiology using a fully automated, multiplexed recording system. Receptor Channels. 9, 19-28.

### Legends to the Figures

### Figure 1

Nucleotide sequence of 14.296 bp spanning the *Xenopus tropicalis Lef*-*1* 5'-flanking region, exon 1 and part of intron 1. The major transcription start site is indicated with an arrow. The first ATG of the coding region is indicated in bold, coding sequence is underlined. The part of the 5' UTR used in the construct is in italics. The HBgl4.5::GFP construct starts at 1278nt (indicated in bold and underlined) and stops at nt 5402 and includes part of the 5' UTR. The EBGl920::GFP construct starts at nt 4497 (indicated in bold and underlined) and stops at nt 5402.

### Figure 2

Genomic organization of 5' sequences, the first exon and first intron sequences of *Lef-1 in Xenopus tropicalis.* Exon 1 is represented by a filled rectangle. The 5'UTR is indicated by a striped rectangle. The length of the different fragments used for the constructs is indicated. The regulatory elements as revealed by the analysis of the expression patterns exhibited by the deletion constructs are indicated underneath the arrowed bar.

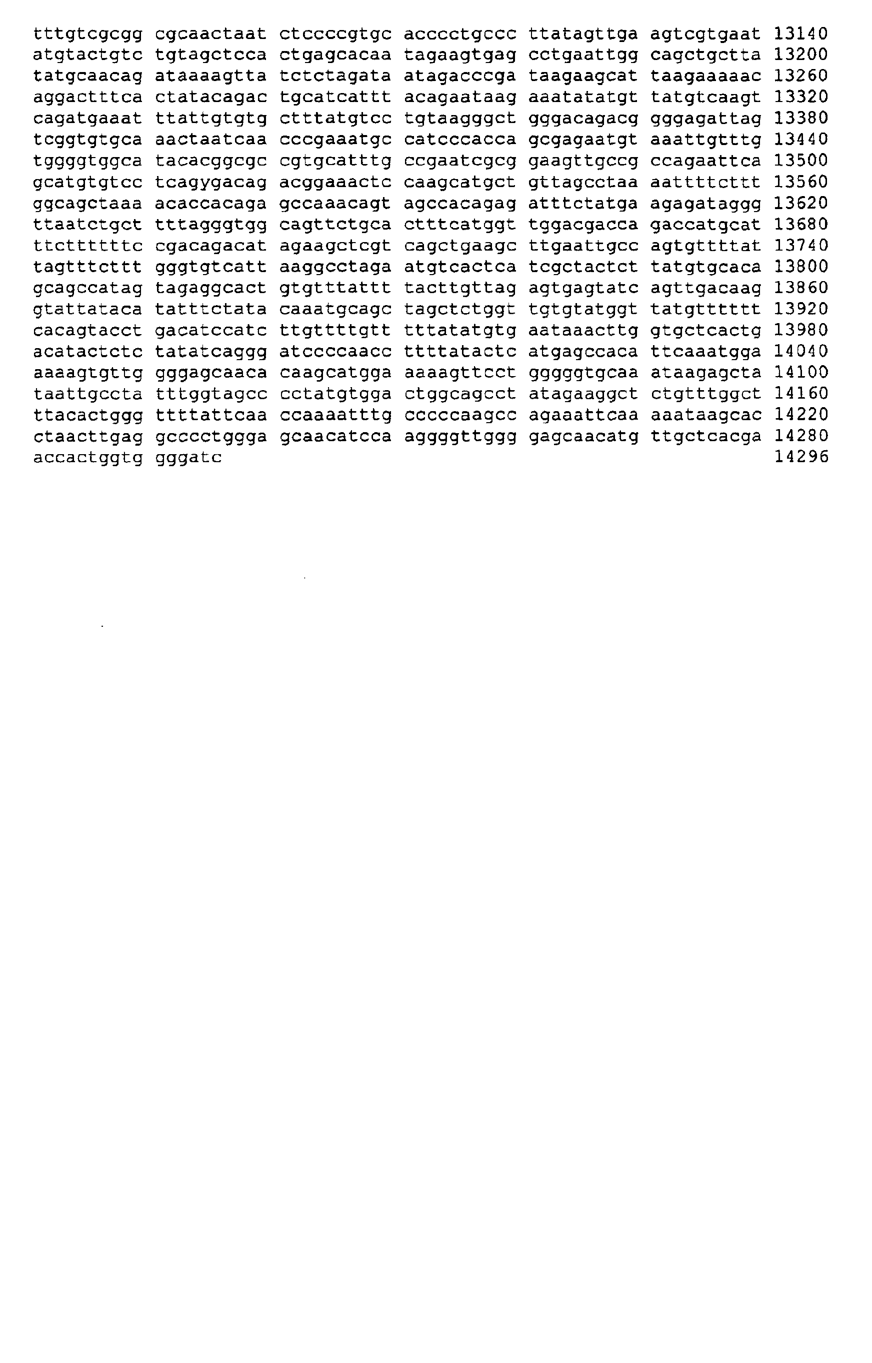

## Claims

1. An isolated or recombinant nucleic acid comprising regulatory sequence of a Xenopus *Lef-1* gene or a functional part, derivative and/or analogue thereof.

2. An isolated and/or recombinant nucleic acid according to claim 1, wherein said regulatory sequence comprises a 4974 nucleotide region located upstream of the transcription start site of said *Xenopus Lef-1* gene, as depicted in figure 1, or a functional part, derivative and/or analogue thereof.

3. An isolated and/or recombinant nucleic acid according to claim 2, wherein said regulatory sequence comprises a 5968 nucleotide region located upstream of the translation start site of said *Xenopus Lef-1* gene, as depicted in figure 1, or a functional part, derivative and/or analogue thereof.

4. An isolated and/or recombinant nucleic acid according to claim 2 or claim 3, wherein said regulatory sequence further comprises a 994 nucleotide 5' untranslated region, a 219 nucleotide exon 1 and/or a 8109 nucleotide intron 1 or a functional part, derivative and/or analogue thereof.

5. An isolated and/or recombinant nucleic acid according to any one of claim 1-4, wherein said regulatory sequence comprises a deletion of an activator sequence, wherein said activator sequence in the context of said regulatory sequence is capable of activating expression of an operably linked of *Xenopus Lef-1* gene and/or a gene of interest in the inner ear, the pituitary gland and/or the pineal gland, limb buds, lateral line system, epidermal hair cells, proctodeum and notochord.

6. An isolated nucleic acid according to claim 5, wherein said deletion is, in the sequence of Figure 1, located between nucleotides 4497 to 5402.

7. An isolated and/or recombinant nucleic acid according to any one of claims 1-6, wherein said regulatory sequence comprises a deletion of a repressor sequence capable of repressing expression of *Xenopus Lef-1* and/or said gene of interest in the forebrain and/or lens.

8. An isolated nucleic acid according to claim 7, wherein said deletion is, in the sequence of Figure 1, located between nucleotides 1278 to 4497.

9. A method for regulating expression of a gene of interest in a cell comprising providing said cell with said gene of interest in operable linkage with a regulatory sequence according to any one of claims 1-8, and culturing said cell to allow expression of said gene of interest.

10. A method according to claim 9, wherein said cell is a *Xenopus* cell.

11. A method according to claim 10, further comprising producing a transgenic *Xenopus* animal from said cell.

12. A method according to claim 10 or 11, wherein said *Xenopus* animal and/or cell is *Xenopus tropicalis or Xenopus laevis.*

13. A method according to any one of claims 9-12, further comprising providing said cell and or transgenic animal with a compound and determining whether expression of said gene of interest is affected by said compound.

14. A method according to any one of claims 9-13, wherein said compound comprises lithium.

15. A method according to claim 13 or claim 14, wherein said compound comprises a proteinaceous molecule or a nucleic acid.

16. A method according to claim 15, wherein said cell is provided with said proteinaceous molecule by providing said cell with a nucleic acid encoding said proteinaceous molecule.

17. A method for directing translation of an mRNA in a cell comprising providing said mRNA with the 5' untranslated region of a Xenopus *Lef-1* gene or a functional part, derivative and/or analogue thereof.

18. A method according to claim 17, wherein said mRNA comprises at least two coding regions.

19. A method according to claim 18 wherein said 5' untranslated region is located downstream from the translation start site of the coding region closest to the cap-site of the mRNA.

20. An isolated and/or recombinant nucleic acid comprising the 5' untranslated region of a *Xenopus Lef-1* gene or a functional part, derivative and/or analogue thereof.

21. An isolated and/or recombinant nucleic acid according to claim 20, further comprising at least one coding region for a gene product.

22. An isolated and/or recombinant nucleic acid according to claim 20 or claim 21, wherein said nucleic acid encodes a poly-cistronic mRNA.

23. An expression vector comprising an isolated and/or recombinant nucleic acid according to any one of claims 1-8, 20-22.

24. A cell comprising an isolated and/or recombinant nucleic acid according to any one of claim 1-8, 20-22 and/or an expression vector according to claim 23.


SEQUENCE LISTING

<110> Koninklijke Nederlandse Akademie van Wetenschappen

<120> Modulation of Lef-1 expression and modulation of
      regulatory region for (partially) mimicking Lef-1
      expression

<130> P65171EP00

<140> 03077243.8
<141> 2003-07-16

<160> 1

<170> PatentIn Ver. 2.1

<210> 1
<211> 14296
<212> DNA
<213> Xenopus tropicalis

<220>
<223> /note="14.296 bp spanning lef-1 5'flanking region"

<400> 1
cagctggagc tccccgcggt ggcggccgct ctagaactag tggatcccca accttttata 60
ccagtgagcc atgttcaaat ggaaaaggtt ttggagacca gcacatgaaa aatgttcctg 120
gagatgccaa tgagagctat aattggctat ttgatagcct ctatgttgac tatgaagccc 180
acagaaggct ctgtttgcca attacactgg gtttttatgc tatcaaagct agcctcctaa 240
ccagaaattc aaaaataagc acctgctttg aggcccctgg gagcaacatt caaggggttg 300
gtgagcaaca tgttgctcat gacccactgg ttagggatta ctggcctagg ataaggccag 360
agacacttag attacacatc cctggtctca agcccacctg aaagatatca tttacaacct 420
ttttatgcaa actgttttgg agagcaaata atttcacaat gtgggatcat acagcacaaa 480
aaattatgaa gacttgtcct tttgaagatg ctaaaaacca aaacattcat actaaatttc 540
aacaagctat tatgtatgca ttagtactta cctatttagc ttctttgtgt tttacagtag 600
ctgattctag acagtccata caatcatcat ttgggttgca tttgatacaa tagaaataag 660
aacaggatat ggcatgagga tatatgcctt tgctgaatgc caccaaccag atctctggat 720
ggaaatcaaa tggaaggaag gctgattatt atagcaatac tattttctct cacagtacag 780
ttttgcgtct ttatacttgg gtttgtagga atcctattga tggttctctg tcatatatga 840
ttatttagaa attatttgta gcccatatat atattatggt ggtaaatcag gatggcatta 900
taaaaggggt ggataaaata tggaagcatt gtgcatttgg ttttggtgtt aatatataag 960
agatactatg tgagttgcta aatattctat tggcatttcg ttttaagcct tatagcacca 1020
gtaatgtcaa gaactgtgcc ctgtacaagt tagtttttat tactctcagc caattcaagg 1080
ctaggggaat aatgctgctt ctagccatac tagttctgca actacccgtg aggaaaaaga 1140
ggcagcttag cctcaccatg taactattgc cactgggtgg gaggttgatt ctggacatta 1200
ttaattttta gtaaatctgt aagaacatct ataagcatac ctgttgtaaa actttttctg 1260
ctaggaacat ggtaaccaag cttgatgttt aaccttaatg acccaaccta gtattgttaa 1320
caaaaacaaa aaaccattgt gctctgcctc cattgtaagt agctaatggg ttctaaatat 1380
ataaaacatg tctttacata cagagaaata aaccagtaac tgttttgtat taaatttaaa 1440
tagcaattct accaattatt ataaatatgc agaccacaca ctattcaata ttagatgttt 1500
aattgaatca tctaactggt aataattgcc ttgttattgc tttatgaaac gtaaagctag 1560
gaaatacagt aatgttattt cctacggtgt cctatttaga aaaataaatc ttaatttctg 1620
actaattaaa catttctttg taataagaca gcaacttgta cttgatgttg actaaacctg 1680
tatatcaata cactgattgc taaatattac tattgtgcat atctaatgtt taaatgtttt 1740
tagttcccct aagggacagt gctttacatt atgaaaagat ctccttatct ggtaaaccct 1800
aggtcatggt aatagatccc atgcctgtgc tagtgactaa aggtttgata ggtaatagag 1860
cacaaaagca aatctgatga attatgtatc tctgctttct gtaatttcag tatgtatata 1920
tggctgtgtg aaatatacac acttcatgta gaactgaaaa atctatgctg ccactttaat 1980
tatgttaaat tcagtgttag aaataaatta tatacaatgc atcattaaaa caatcaaggt 2040
tgttttaata gagtacaatt gaaaaaaaat cattttataa aaatgttgta caagaagcat 2100

agacttctgt tctgttttaa tcaaaattga aataatcata taaatctgtt gttggcattc 2160
acaaaataaa ggctgactat ataaatatat taatttttct ttaaataagc cgtggttatc 2220
atgaattagc tcgtgctgca gtagaaaaca gttcaatttc atggagtagc ttcacccaca 2280
aacgaaatcc gtgtggatcc gtttcctttc ctgcttcttc ctgttctatt ctcctttatt 2340
aatttattct ccccatcccc aaatattgta aaaaaccacc tgcaggtttg tgtgttttta 2400
cagtcaagct agataaggtt tccctttcct tttttataaa tcatatgtcc ccttctcccc 2460
agcattctat atgttaggaa aataaagaat ccctctgcaa aaaatctagc ccacatactg 2520
taaaagagtt tagatatagg ggaggtgcta atcctcgaat ttcattaaaa ttggtttggg 2580
aaagggaaat tggattgcac taagcaaaat ccacactcca agctaccaca acttccccta 2640
ccccaaatat ttctaaaata aattctgttt tagacaatta attgtctaaa taaaaagatg 2700
ccttgggaaa accctctgac taatactagt ttctgtgttt aaagaaaaag gtttaattct 2760
tatcatatta aaacccagcc agtgtagcaa atagagattg cagtaaactg gatcttcatt 2820
tccatgcttt gctacaatga gataaaataa taatatttat gattatataa ctataagtgt 2880
tgtacccaca ctcacttctt gccatgtaat tctcatactt gcatattctg gtgctgaaat 2940
aaattgcatg aaatgcaaaa taaaggtttt atgttttcat gatctaatcc atcctatata 3000
tttgtgcgac atttaatgat ataacttcac atgtacaatg cttttatcaa aatatgcttt 3060
aaatatcaac ccgaacatta gactaatata tacatatgta cagagtgttc ttcccaagca 3120
acatttttat tgtttatttt tcctttgttt tgttgttttt ctttcttttt attttgctaa 3180
ttctactgag tatttagaag gcaataaaag gaggatctga cggtcttcct gcagtctgta 3240
tgaggatttt agtatctgca gtctatttat taccaacgtt gacaaaacaa tcagacaggc 3300
ttccactaat tattctatgc atttattgcc ctatctcact gcctccaatg aagtctagga 3360
caagaggaag gtgctctaaa aaattagtat gtaaatccct gcaattagca tgaggaagaa 3420
aatgggattt gcattggcaa taggtgggat cctgtaacaa tgtatcacta acttacaaat 3480
cacttgccta tccatcaagc catcatgtac aggtttaaag cccacttctg caaagaacaa 3540
taaaacactt tcatttcttt ttaacattat tgggtggatt acattataga tacaaacact 3600
ataaatcatt aaaaactaaa atgactttaa aataaatatc catacatatg tatctgacac 3660
ctttaaatgt gagtaaatta tgttatatta gtagtattag cataaaattc aaattcaagt 3720
attttctcat tttaatagga agcaagtgat atttactaat tcttgtaaac cgtagcccat 3780
ctatatgttt ccctgctgct aaaaatgtga aaagatcaaa agatacaaag aatgcctttt 3840
gtgtttgtct taggcacttt tgtgtgctga atttgtgcag ttggtccaag tgaagagggt 3900
tcaataaata tactcactag cagatcccag gtgtcttttc cattatataa ccagcctcct 3960
tcttctctcc ttgtttttct gtggatcttt acccggtact caggaaggtt ttctctttcc 4020
catctatatc catcattcag atggggtctg tatggtgtta tttctgtttg atctcagctt 4080
ttcctcacat aagctctcct gctgcatgcc tctggggtgt cgggatatta tagcagggat 4140
atgacaataa cacccattgc atatagtata ttatatatgg gttcttttat ttttatatac 4200
atatatatat atatatacat acattcatac acaaagtatt tatacacaca catatatgta 4260
ttctccttca aaatggctgt cctctcagct gttgctgaac tacagttctc agcatccttt 4320
aaaaggatgt tggtagttaa caagaaacta aaagtcccaa agggaacaca cctatacagc 4380
atttttttcc catatgggtt ttctttacct taaatcaaac tattactacc ttcagtactt 4440
tatttctgag gtcataaatc ttttatgtaa aaagaaagaa aacctaattt tatacagaat 4500
tctgacaatg ctaaatgatt tattattatc actgatgtta gcagagacag gaaaggaaat 4560
aatctggtta gtaattgtac ccccagttga tgtttagttg cacttacccc cacccctccc 4620
ttttaaactc cagaacaaaa agaaatcttt ttctcttccc cttgacaagt acacctgccc 4680
tacttattag ggctttccac tctgtttttg ttaattgcta ttattaggat tatttcattt 4740
tggactattt gcctccccct ctctcccttc ccctcctgcc ccttcattta cagagttgca 4800
ttatgtgtgt gtgtgtgagt ctgtttccct cctctatcag caagctgtca atcaccgcgg 4860
cggctctctt tcatctcttt ccaccgcctc ctcccctcca gcagacagaa agagggaggg 4920
aggggtggaa ggagagacag aaagaggggg gcacaggagg agggggatgt taaagttaca 4980
ttacacaaac acacactgcg tgccccttca ttgagagcaa gggacggcaa tgtgcctctt 5040
gcagtcactg aggtaggaca agacttttgg gagagtttta cgtggtgtga agaaaggaga 5100
ttaacttgtc cccccctccc caggattttc tcagaggtga ttttctcaaa gtgacgctaa 5160
acagaagaag gtaggatcgc tggcaaaagg tacattccac ggggggtttg tggacaggtc 5220
cctttgcaga ggatatgcag cgctttgctt gtcctccctc tgtacccggt acagaccgca 5280
gttttacctg agggtccaga ggaggagttc agtccgctcc ttgccaaaag ctcacggcgc 5340
tctccaaact ttgctcttac ttgcattgag ctgactggca gtgctagcag gacaaagcca 5400
ggctggctgg ggtgtgtggg caccgcacag agagtggcta gtttacctcg cttatttaaa 5460
tgaagacatc ctttctgggt tagtcatctc tgagtcactg actgcataaa gcaaaaaacg 5520
taatttgcag ttagtcagaa ccataacaaa cactgcattt tctcactgct cctcgtgtga 5580
ctgcacacac tcgcctgaat gggagcagaa tgagataaaa gcccatcagc tgtgggtggt 5640
tttggatact ggtgactaca gtagcccccc tggaccaggg gagacaggat tggagccctt 5700
ggagcagagc agagacacac tgggacttgc agccttggga gacaggggtg aactttttac 5760

aagcaaaagt gtcttcttat caagatttgt gactttattt tgctgatttg ggggcaaaag 5820
tgtccagcca gtgccatcag ttttggcatt ttgaccttgg atcattgggt gtggaaattt 5880
tgggaaggga acctttgaat tgggttttgt tttttttttg ctggtgtttg atttctgacc 5940
tgtaacacag aagtcctgat ctggagccat gcctcagctc tctggagcag gggggggcaa 6000
tgggggtggg ggagatcctg agctgtgtgc cacagatgag atgatcccct ttaaagatga 6060
aggggatccc caaaaggaga agatctacgc tgagatcagc aacccggagg aggagggaga 6120
cctcgcagat atcaagtcat ctctggtcaa tgagactgag attatcccca gtagcaacag 6180
tcacgaggta agcaatggaa ttcatttata gcggtaatcg tctgtgttta taactaagaa 6240
cacagtttgg atttttgttt ttgcaacaca atccacaact tcttcaactg tttcctttta 6300
aagtcacatc aacattgcaa aacgatatag ctcacatatg attccatgtg ctgcctttca 6360
aaaaacaaaa aactttttat catccagttt gtcacattta atgggatacc acacaatgcc 6420
catgcttgac ttaatgatag agattccaaa agattcacac gatagtggca gggaagcacc 6480
atgcaattat ccacaactgg ttactttacc ttatgccaac ataatattca tagaagtgac 6540
atgaactact ttctctggct gtaaggaatc cagattggga tgtgcatgca gaatattctg 6600
taacacacag gagtctagat gggtggtact cttttatgag ccttaactta ttttgttttt 6660
gtgttatact actgattgaa tccagccact tttgttttaa gcaccttttt gtgtttgcaa 6720
tgttttgtaa gaaaacctaa ccttttttgt aagtaaactg ctatttcagg ttgctcaaaa 6780
tataatattt gatgccacag gcaaaaaaaa accccaaagt gacctgcctc agttcacatg 6840
atatttgatc agggattaaa gactcaaaga cccctaggaa aacagtccca ggtcatgtgg 6900
gggctctcag tgtttgagct ttttccgtag gctacactaa ctgcctgagc ctacaataaa 6960
tcttttctag aggtatattc aatatgccca gttcaatgaa taaacaaaag cagaggcact 7020
gatgccttta aatctctgaa gttagttcat gacacacact taattggcat agttttttgt 7080
agcttaactt gtgtctcctt ttaacaattt taaagatcta gctggagaca tccaggctgg 7140
taagaaaaca ctctgagcac aattatacag tttcagcttc aacatcttat tgcccgacat 7200
gttctcttga gtggaccaag atgaagcatg cagacattct gtgttttttt caacaatgat 7260
gaacctgcag ccctctagct gttctctccc taaaaactgt cattatcttt gtgctcagat 7320
gtttaactga attacttcag ttaggcttcc cccccacata tatctttaga tatgaaagga 7380
tgaaatgggt agaaaataca tatcatatca aatcgcttga agttaattga aaagttgcag 7440
aaattacatt taagaattgg taaaaattaa aagtattagt gattctattt aacatttgca 7500
agaataatga gcaagttgtc accaatcaca catgcacttt gtaacaatac aaaagggttc 7560
caggctagca aatgacataa cgttttctgc ctatgtagag gaagtggcat ttgtgttaga 7620
aaaggtgtaa gtgatacaga ttttaacttc ctccaagtca ctctattggt gcagtcatgc 7680
agtttacagt agtagaaaat gtatatttta gctggaaaat atattttgta aatataccag 7740
tgggattcta attgctacaa ctacctttat tcctacttac attgtacaat agtactagtc 7800
agaacattgt gcacacaaag gagggggcag aaaaatcatt gttttccttc tatagtattt 7860
gtactgaaaa aaatccacct tgcacttaca taatgaattt gtgttacgaa aagaatatat 7920
taagctaaac caaaacagga tgtctgggaa atgattctta tagctgtgat acataaaatt 7980
aatcagggaa tgcgaaaatg gtttgttaca aaaccactgc agattaatct tatttataca 8040
ccttagtatt tttatcatca ttgtctgctc tgtagagtca catttctgta ttacaaatat 8100
gtacagatta tggaatccat atcagtggca aaacaatcct attgtgttta tttaatgttt 8160
aaatgatttt tagcagacta aagtcatggt gacctaaatt acagaaagac cccttgtcca 8220
aacagcccca ggtcccaagc attacagata atggatccta tacctgtacc agtttgtaaa 8280
tgacagcaca cagtcttcta tgacagacaa aacacagttg tttacattgt ttgaagctgc 8340
catgcataca ttgcaataga tgaagtactc agaagtcaca tcagagatca ctgacctgaa 8400
tgttgtgtct ttgcctgctg ctttgtgctt ccatttagtc actctttagt aacatctatt 8460
tttcagacta cttacagtaa ctttatcagt actttaactc acagacaaat atttaacggg 8520
atactgttat gacttgttag gcctcagcac accttgtggt ataacttggt actctcaggg 8580
agacctgcag cggtcaaaac aacctttaat tgtttatctt tacttcctat ggacatgcac 8640
tggacatgct ctgcctatgg gaaacattaa tggtgacagg caggcacaag tttgggacac 8700
ttgcttctcc caaagataaa tgtcgtgatt gtaacaccat gtgtatcaca gtccaaaaaa 8760
gaattattta tcttcctcgt gataaagcat tacctgcatt cagctgtaac tgaatctgag 8820
ctggttgtga gcaatatgtc tttctataag ctataccact aatacttgca aaggtgcaga 8880
ggatgtatga tgtctaccta aaatgatttt ttgcagtgaa aggatgtaat tttaattagt 8940
tttccaatat acatttatta aaaatgctca gcagtttcaa tgttatttgt acattttatt 9000
actattgaaa atgtcatttg tgcctcattt tttttttttt tgcactgttg gttctgactg 9060
ttgaaacagt ctagctagca gaagctagtg gcttaacagg cattgtttca agtgtcacaa 9120
acataggact gaaaaggaaa gggataaaca aatactgatt tcagttaaat tacatataca 9180
caataaaacc atggaacatt tttagttaat acatattgga aagttgttta caaatgtaat 9240
ttaatatccc attatttgaa tatctatact atacagagtt gacttgtagg tcagtgcttt 9300
caaaactgta ttgctaaccc ctgagtggta cagagctagg gggccagcct aaacgtcagc 9360
tagggtgaga tttggggaaa aaagcttttt tttctgttat agcctcttga gagctccctt 9420

aaatgtcaag tatggagata gattttgtga aaatggctga atgaatgcaa tattttcata 9480
tatccatatt gttcctgtga gtgtaggagg accttgacca aggaaaactt gaaccagaaa 9540
aatcagaaaa ccaaggaaaa cttgtgcagt tggcaacagt ctgtgtctga gtgtctatgt 9600
aatccttgcc cttttgttaa attgctaatt gctgtcctga attacaaaac tcagtgtttt 9660
cattatgatt cccaacagta ggaagggctt cacaaagact gtttattgac tggagtctct 9720
cttatctttt gctgttaggt gtctgctttc ccttagctgt atgttaatta ttctaatgca 9780
tatctaatct tagcgtaacc ttttcatgtt tcagcttccc accagccctc tgaatttcgt 9840
aaaactcatt tatttgttta ggccagctca tttagggaaa ttgcttctaa tacagagact 9900
tgagattttt tgtgtgttga tgtgtgcatg tgttttggca tgtgtgctag acctgacatt 9960
cacctttggg ctaaggtcac actggctgac tttttatata agatttgggt caatgacaca 10020
ctgggcattt atcctctgca taaaatcaca ggcaacaaaa cacctgaaaa tgccatacca 10080
ttgaattgtg ttgaaatctt acatgtaatc atgccaaaat aaccacagat gcatttgcat 10140
gtgattacat gcttttactg acacacttgc aacaatttaa atgcaataca agggcatgtg 10200
tgccacaagc acacatgcca gctgctttac acttcctgaa atgtgtgtgg tgacaggctt 10260
gggatcagcc cttaagtata cacacagaga tgtcatttcc cctggttttt ttagagggtt 10320
accattttag agcacctccc ccagcatcct gtcactatat ggtaatcacc tggttttgaa 10380
atctcatagt ataccttaaa gatgcccatg cccagtgagc ttcttttcag tggaaaatga 10440
tttgtgtatg tgcagtgaga actgagtaac gtcatagggg ctttattgca ttgcacagtg 10500
atgtcaccag gtccactgca attagccaca cccacagttt tcagcaacgc tgcagttccc 10560
ccctggatgc acatcattaa ggttgacatg cacacacaca gtgaatttca gctcagcaac 10620
actaaagtat gctgaaattt gcgaggtgtg tgctgatggg ctgatccatg cctgtcagct 10680
cacacacctt cacgaaaaaa aaggcagcag atcatattat ttcactagta tatatacacc 10740
atctgtctgc cattagcctt agttcttaca aaaaaaaaat caggaaccaa atgccatgca 10800
tgactctttt agggttgcca cctggtcctt tttgtacccc cccaaaaaat ggcctaggct 10860
ctttacagga cttccccccc cccccccccc aaaaaaaaat tgcctaggct ctttacagga 10920
ctgtatagac ctttgtcata aagaatagga catgtgttgc caagaaagtt tgctgtagtt 10980
gtctaatcaa cagatacaaa cttacaaaca gatacacact gcttcccatt caaaaatacc 11040
tactgcctcc cattcaaatg gaagacctta acaccaccac tagtattttt tgaccagaaa 11100
aagtgcagca gaggacaatt ggaggtggaa ggtgtggttc aaggtgcttt tgcatcggat 11160
gaaataaggc agtgatgaaa acacccagta tggcattagc cttaggtctg ttgtacaagg 11220
aggcaccttt tcctctgaag tgattatcta tatgcagatc tcttcaggta gtacatatta 11280
ttgacttcaa agctaaaact ggtgctgcca ccttcactga aaatgccagg catcccttgc 11340
aaaaattaga ctcgagcaca ctcaattttt actcttttgt gcaactctta aatctagaga 11400
gggattcctg gaagatgcta agatggtggt gtggcatttg cttagaaaat tcccctgtgc 11460
taaagcaatt gttttccaaa aggaacactt ggcctgtgct taatattttg gcaccccgta 11520
ctgaaatagg gtgttttacc tgcgctttta tttgtttttg gaaaaaataa actttcaagt 11580
tgccaatgct ttatttgtcc acactacata tttatatatt atattaaaaa aaaatttgct 11640
gcatgactac caacccatat tttctgtgac cagtcatatc tccacaataa cagtctatgg 11700
ggctgattca ctaaaggtcg ttaaaacgag cgctatttat agcatgcatt aaaaatttta 11760
ttgcttctta tttttcacga tttaacgcac aattcactaa aaggatactt gcgttaattt 11820
agacgcgatg ttgtttgttt gcgttattta agtcgcactg agtgtttttc ttgtattatt 11880
ttccgcgcgt gtgttaatta tcgctgcgcg cgatatattt cgccgcttgc tatattggcg 11940
cacgatttaa agcacgtaac cattaggttc ataaaactac ttttctgaaa atgaccattt 12000
ccctgcaaac tggaggctgc atgactctag ggccaacaca tacttgaaaa aatcccactg 12060
caaagtccat gttgcgttgc caaaagctca ccaaccacaa ttttcttcaa gtaccgcctg 12120
ccccaagtag gtgttaatct ttgcacagac taatgcgata tttagcgcgt ttaacgcttc 12180
atatgtgttc gtaaatcatg cgttagtatt atttctacgc gcgaattaac gcagtgtgtt 12240
aaagttaacg catttcagta gcgcacgcat gtgaaataac tttgacgaat cggcattatt 12300
tatcatgtca attttaacgc aaaaaaccat gtgatacccg cttatcgacc tttagtgaat 12360
cggccccttt gtgttaaatg acttactgat ttattggyyt atataccaaa gcaactatcc 12420
tttaaaggag aaggaaaggc taagtcactt gggggtgcta aaatgttaag caccctcaag 12480
tgactttaat cgcttacctc gtaccccggg ctggtgcccc tgttaggaga aaacagcacc 12540
agcccggggt acctggagca atgcgcttcc tctttccacc tgcgtttcgc taagactaac 12600
acaataggcg catgcgcagt agagtgaaaa gccgacttct ctgttaaagt acggcttttc 12660
actctactgc gcaaagcagg aagaaggaag cactgcagct accccgggct ggtgctgttc 12720
tccccgtaca ggggcgccag ccctgggtaa aaggtatgcg attcaagtca cttaggggtg 12780
cctaacattt tggcaccccc caagtgactt tgcctttcct tctcctttaa ccagttgtac 12840
aataatatct gtgcatagtt agttttagtt ttagggcagt aacacacggg gagattagtc 12900
gccacgcaac aaatcgttgt cgcaggcgac taatctcact gcagtgccat cccacctgct 12960
agaaagtaaa tcgctggtga gatggcatac atggcgccgc gtatgccatc ccaccagcga 13020
tttacattct agtcggtggg atggcattgc ggggagatta gtagcccacg ataacggaga 13080